Europäisches Patentamt

European Patent Office

Office européen des brevets

(11) Veröffentlichungsnummer: **0 307 363**
**A2**

## EUROPÄISCHE PATENTANMELDUNG

(21) Anmeldenummer: **88810607.7**

(22) Anmeldetag: **07.09.88**

(51) Int. Cl.⁴: **C 12 N 5/00**

(30) Priorität: 08.09.87 CH 3461/87

(43) Veröffentlichungstag der Anmeldung:
15.03.89 Patentblatt 89/11

(84) Benannte Vertragsstaaten:
AT BE CH DE ES FR GB GR IT LI LU NL SE

(71) Anmelder: **Solco Basel AG**
**Gellertstrasse 18**
**CH-4052 Basel (CH)**

(72) Erfinder: **Miltenburger, Herbert G.**
**Hobrechtstrasse 15**
**D-6100 Darmstadt (DE)**

**Baschong, Werner**
**Maiengasse 27**
**CH-4056 Basel (CH)**

(74) Vertreter: **Frossard, Michel, Dr. et al**
**A. Braun, Braun, Héritier, Eschmann AG, Patentanwälte**
**Holbeinstrasse 36-38**
**CH-4051 Basel (CH)**

(54) **Verfahren zur Vermehrung und Haltung von tierschen Zellen in Massenkulturen.**

(57) Durch Zusatz eines von makromolekularen Substanzen freien Hämodialysats oder Ultrafiltrats aus Kälberblut, dessen Komponenten Molekulargewichte unter 10'000 haben, können tierische Zellen und Gewebe sich in Massenkulturen vermehren oder gehalten werden, ohne die Schädigungen aller Art zu erfahren, welche die in vitro-Situation (zellulärer Stress) bewirken kann. Es werden vorzugsweise 0,5 bis 4 Volumen-% Hämodialysat oder Ultrafiltrat, bezogen auf das Volumen der Massenkultur, zugesetzt. Zudem lässt sich dadurch das bei Zellkulturen bisher unerlässliche tierische Serum ganz oder teilweise ersetzen; die mit dem Serum einhergehende Einführung fremder Eiweisse kann vermieden oder vermindert und damit die Reinigung biologischer Produkte wesentlich vereinfacht werden.

**Beschreibung**

### Verfahren zur Vermehrung und Haltung von tierischen Zellen in Massenkulturen

Unter tierischen Zellen in Kultur versteht man alle isolierte Zellen oder noch in einem beschränkten Gewebsverband befindlichen Zellen aus wirbellosen Tieren und aus Wirbeltieren (inklusive des Menschen), die unter künstlichen Nährmediumbedingungen als primäre, sekundäre, tertiäre usw. Kulturen oder als permanente Zellinien/Zellstämme kultiviert werden. Beispiele der tierischen Zellen sind die Fibroblasten und Fibrozyten, die Osteoblasten und Osteozyten, die Hepatozyten, die neuronalen Zellen, die Epithelzellen, embryonale Zellen aller Art, benigne und maligne Tumorzellen.

Verwendet werden Kulturen von tierischen Zellen insbesondere
- zur Gewinnung der Inhaltsstoffe dieser Zellen, z.B. Enzyme, Hormone, Lipide, Glykolipide, Glykoproteine, Nucleinsäuren, und von Zellorganellen wie Ribosomen, Mitochondrien, Membranen, Chromosomen usw.,
- zur Herstellung von Zellverbänden, z.B. von künstlichen Geweben auf einem Dacron ®-Netz,
- zur Züchtung aller Arten von tierischen Viren, z.B. AIDS-Viren oder Insektenviren, von Vertebratenviren, von Mikro-organismen und Parasiten,
- zur Herstellung von Impfstoffen, Wachstumsfaktoren und anderen Faktoren mit biologischer Wirkung,
- zur Züchtung von Zellen mit eingeschleustem fremdem genetischem Material, wie infizierte und transfektierte Zellen zur biotechnologischen Herstellung von zellfremden Stoffen,
- zur Züchtung von induzierten Zellen und Hybridom-Zellen, welche zur Herstellung von Antikörpern verwendet werden.

Zu diesen Zwecken werden Massenkulturen angelegt, d.h. solche, bei welchen in der Regel aus einer Einsaat von $10^4$ oder $10^5$ Zellen per ml Nährmedium $10^6$ und mehr Zellen per ml Nährmedium nach einer bestimmten Kulturdauer erhalten werden und wobei auch dies in grösseren Nährmediumvolumina erreicht wird. Vielfach arbeitet man in Suspensionssystemen (Spinner und Fermenter ab 0,5 Liter Inhalt, wobei bis zur Grössenordnung von etwa 20 bis 25 Liter Inhalt noch von "Laborfermentern" gesprochen wird. Die industrielle Fermentiertechnik beginnt jenseits dieser Volumengrenze und erreicht Grössenordnungen von bis über 10.000 Liter. Aber auch immobilisierte Zellen in Röhrensystemen, in Rollerkulturen und auf festen Trägern einschliesslich Microcarriern können als Massenkulturen Verwendung finden.

Es ist aber bekannt, daß tierische Zellen in künstlichen Kulturen nach mehrmaligen oder häufigen Überimpfungen ( = Passagen) schnell degenerieren können (Ullmanns Encyklopädie der technischen Chemi, 4. Auflage, Band 8, Seite 501, Verlag Chemie GmbH, Weinheim/BRD 1974). Ferner können im Verlauf einer Massenkultivation Schädigungen der Zellen auftreten, welche einerseits die Folgen verschiedener Manipulationen darstellen und im Grunde mechanisch bedingt sind, andererseits durch Abweichungen von den für die betreffende Kultur erprobten und optimierten Standardbedingungen und im Grunde auf chemischem Wege hervorgerufen werden.

Auf welcher Art die Degeneration oder die Schädigung der Zellen auch zustande kommen mag, sie hat jeweils als Konsequenz eine Herabsetzung der Qualität der Zellen und der Erzeugung von biologischen Produkten (Verminderung der Ausbeute). In der Tat werden immer wieder in Massenzellkulturen Schädigungen der Erbsubstanz, der Zellmembran, der Zellorganellen und anderer Zellbestandteile, Mitosestörungen, ferner Schädigungen, die zu unerwünschten Zellzyklusänderungen führen, aberrante Stoffwechselleistungen oder Störungen der bioproduktiven Leistung sowie ganz allgemein letale Schädigungen festgestellt, welche die Wirtschaftlichkeit der Kultivation in Frage stellen.

Als Beispiele der zellulären Stress-Situationen, welche auf chemischem Wege zustande kommen, seien genannt die Verarmung des Nährmediums durch Aufbrauchen der Komponenten, der Austausch des Nährmediums gegen eine frische Lösung, der Austausch bzw. der Ersatz einzelner Mediumkomponenten, der Carbonatentzug bei kontinuierlich durchgeführten Kulturen als Folge einer nicht sofort behobenen Panne des $CO_2$-Zufuhrsystems und/oder teilweisen Carbonatzersetzung im Medium durch Hitze oder Säure, die öfters angestrebte Herabsetzung des Zusatzes von tierischem Serum. In der Tat ist die Herabsetzung von Serumanteilen oder eine völlige Serumfreiheit in Nährmedien für tierische Zellen in solchen Fällen wünschenswert und anzustreben, in denen die zellkulturen (Monolayer oder Suspension) zur Gewinnung von Bioprodukten verwendet werden. Solche Bioprodukte müssen - insbesondere wenn sie in der Humanmedizin eingesetzt werden - frei sein von Fremdproteinen. Bei Verwendung von tierischem Serum im Nährmedium sind daher aufwendige Reinigungsprozesse erforderlich, um das Produkt proteinfrei zu machen.

Als Beispiele der zellulären Stress-Situationen, welche mechanisch bedingt sind, seien erwähnt der Sauerstoffeintrag durch Einblasen von Luft/Sauerstoff ins Nährmedium, das Rühren des Mediums in Spinner-Gefässen oder Fermentern (Bioreaktoren), das Umpumpen der Zellen oder des Nährmediums ohne Zellen von einem in einen anderen Behälter oder durch Kapillarsysteme usw.

Die Schädigungen von Zellen in vitro durch technische und kulturbedingte Manipulationen, z.B. in gerührter und mit Gasblasen zum Sauerstoffeintrag durchperlter Zellsuspension, entstehen dadurch, dass hydrodynamische Effekte (Scherkräfte) auf die Zellen einwirken. Solche mechanisch ausgelösten Stress-Situationen sind seit langem bekannt und in der Literatur eingehend diskutiert. 1979 beschrieben Katinger, H.W.D., Scheirer, W. und Kromer, E. (German Chemical Eingineering 2, 31-38: "Bubble column reactor for mass propagation of animal cells in suspension culture") die zellabtötende Wirkung zerplatzender Gasblasen an der Oberfläche der Suspensionskulture durch Scherkraftwirkung.

In einer Veröffentlichung von Cherry, R.S. und Papoutsakis, E.T. ("Hydrodynamic effects on cells in agitated tissue culture reactors", Bioprocess Eingineering 1, 29-41; 1986) wird anhand biophysikalischer Kalkulationen festgestellt, dass in gerührten Suspensionskulturen von tierischen Zellen bedeutende Scherkräfte auftreten, die nicht nur subletale Schädigungen induzieren, sondern in Abhängigkeit von den in den Suspensionen erzeugten Turbulenzen auch Letalschäden: Die Zellen sterben dann ab.

Croughain, M.S., Hamel, J.F. und Wang, D.I.C. zeigen in einer Arbeit (veröffentlicht in Biotechnology and Bioengineering XXIX, 130-141; 1987: "Hydrodynamic effects on animal cells grown in microcarrier cultures"), dass zunehmende Rührgeschwindigkeiten ab 60 Upm auch zunehmend zum Absterben von FS-Zellen führen, bis zum völligen Zusammenbruch der Kultur. Gleiche Effekte werden auch für Vero-Zellen gezeigt. Als Ursache werden ebenfalls Scherkräfte nachgewiesen.

Zwei weitere Berichte von 1987 (Schürch, U., Einsele, A., Kramer, H., Widmer, F. und Eppenberger, H.M.: "Influence of shear forces on proliferation and antibody production of hybridoma cells grown in mass culture"; Proc. 4th European Congress on Biotechnology, 3, 569; 1987; Wudtke, M., Kretzmer, G. und Schügerl, K.: "Investigations on the influence of physical environment on the culturation of animal cells"; Proc. 4th European Congrss on Biotechnology, 3, 272, 1987) liefern durch neue Befunde ebenfalls Daten dafür, dass Zellen in Suspensionskulturen durch Rühren erheblichen Scherkräften ausgesetzt sind, mit der Konsequenz von Zellschädigung und Zelltod in solchen Stress-Situationen.

Scherkräfte führen aber auch in einem anderen Kultivierungssystem zu Stress mit folgender Schädigung: Suspensionskulturen müssen oft von einem Gefäss in ein anderes umgepumpt werden. Obwohl dies so schonend wie möglich ausgeführt wird, lassen sich Scherkräfte durch den Pumpvorgang selbst, aber auch während des Durchtritts durch Schlauchsysteme nicht vermeiden. Diese Belastung der mit dem Medium umgepumpten Zellen führt zum Absterben vieler Zellen. Schon 1971 wurden solche Effekte an HeLa S 3- und L 929-Zellen nachgewiesen (Augenstein, D.G., Sinskey, A.J. und Wang, D.I.G.: "Effect of shear on the death of two strains of mammalian tissue cells", Biotechnology and Bioengineering VIII, 409-418, 1971).

Ebenfalls erwähnenswert sind folgende Veröffentlichungen über methodenbedingte Zellschädigungen:
- S. Fazekas de St. Groth; "Automated Production of Monoclonal Antibodies in a Cytostat", J. Immunol. Methods 57 (1983) 121-136. Beschrieben wird der schädigende Effekt verschiedener Rührgeschwindigkeiten der Propeller in Spinnergefässen mit Suspensionskulturen von 10 verschiedenen Hybridomzellinien: Scherkräfte in den Turbulenzzonen der Rührblätter zerstören die Zellmembran und führen zum Platzen der Zellen.
- M.W. Glacken, R.J. Fleischaker and A.J. Sinskey:

"Mammalian Cell Culture: Engineering Principles and Scale Up", Trends in Biotechnol. 1, 4 (1983) 102-108. Beschrieben wird der schädigende Effekt von Luft in Blasenform, die zur Stabilisierung des Sauerstoffgehaltes des Nährmediums eingeblasen wird: Scherkräfte an der Grenzfläche Luftblase-Nährmediumoberfläche führen beim Platzen der Luftblasen zu Zellmembranschäden mit folgender Lysis der Zellen.

Aus den erwähnten Literaturstellen geht hervor, dass die bei Massenkulturen von tierischen Zellen auftretenden Schädigungen zwar erkannt worden sind, dass aber zur Vermeidung dieser Schädigungen nur gewisse systemspezifische Massnahmen teilweise Erfolg hatten, jedoch bisher keine allgemein anwendbare Methode bekannt war.

Es wurde nun gefunden, dass sich tierische Zellen als Massenkultur unter Vermeidung der oben erwähnten Schädigungen vermehren und halten lassen, wenn der Kultur ein von makromolekularen Substanzen freies Hämodialysat oder Ultrafiltrat aus Kälberblut, dessen Komponenten Molekulargewichte unter 10'000 haben, zugesetzt wird.

Erfindungsgemäss können gezüchtet werden tierische
- Gewebe oder Organexplantate,
- primäre, sekundäre, tertiäre oder weitere Zellkulturen,
- diploide oder heteroploide bzw. aneuploide Zellstämme und Zellinien und
- permanente, gegebenenfalls transformierte Zellinien, welche die Merkmale von adhärentem oder nicht-adhärentem Wachstum aufweisen, wobei die Kultur in den üblichen, aber auch in speziellen, für bestimmte Zellarten oder Zellinien vorgeschlagenen oder verwendeten Nährmedien durchgeführt wird.

Das zur Anwendung kommende Hämodialysat kann nach bekanntem Verfahren hergestellt werden. Beispielsweise werden 10 Liter defibriniertes Kälberblut mit 1 Liter Ethanol versetzt, das 1,5% Nipastat (Mischung von Parabenen) enthält. Das Gemisch wird gegen eine wässrige 10%ige Ethanollösung bei einer Ausschlussgrenze von ca. 10'000 ausdialysiert. Nach Eindampfen unter reduziertem Druck auf einen Gehalt von 18% Trockensubstanz und Einstellen des pH-Wertes auf 7,0 mit zweinormaler Salzsäure wird das erhaltene Präparat einer Filtration unterworfen. Zur Erreichung eines sterilen Präparates wird anschliessend noch sterilfiltriert. Die so erhaltene Lösung kann - je nach Bedarf - mit Wasser auf den gewünschten Trockengehalt verdünnt werden.

An Stelle des oben beschriebenen Hämodialysats kann auch ein entsprechendes Ultrafiltrat aus Kälberblut eingesetzt werden. Zur Herstellung des Ultrafiltrats können bekannte Ultrafiltrationssysteme mit einer Ausschlussgrenze von ca. 10'000 verwendet werden wie insbesondere die entsprechenden Pelicon-, Romicon-, Amicon-, Ultrasart- und SKAN-Systeme (jeweils Markenname; Hersteller: Millipore Co., Bedford MA/USA; Romicon Co., Woburn MA/USA; Sartorius GmbH, Göttingen BRD; Gelman Sciences, Ann Harbor MI/USA).

Vorzugsweise wird ein Hämodialysat oder Ultrafiltrat verwendet, zu dessen Herstellung ein etwa 6faches Volumen von Kälberblut eingesetzt wird.

Vom Hämodialysat oder Ultrafiltrat sollen mindestens etwa 0,5 Volumen-%, bezogen auf das Volumen der Kulturflüssigkeit, zugesetzt werden; im allgemeinen wird der Zusatz etwa 0,5 bis etwa 4 Volumen-% betragen, in Abhängigkeit von der jeweiligen Organ-, Gewebe- oder Zellkultur und von den Kulturbedingungen.

Die erwähnten Gewebe sind beispielsweise Gehirnexplantate, Epithelzellen, Blut und das Knochenmark, während als Beispiel von Organexplantaten Teile der Leber, des neuronalen Systems, der Muskulatur, der endokrinen Systeme, des Gefäßsystems genannt werden können.

Primäre Kulturen sind solche, die aus Zellen bestehen, die direkt aus dem Organismus entnommen und für eine gewisse aber begrenzte Zeit unter künstlichen Nährstoffbedingungen ausserhalb des Organismus kultiviert werden können. Hierzu gehören Zellen aus Haut- und Organbiopsien, Embryonalzellen, Zellen und Zellverbände von adulten Individuen usw.

Sekundäre, tertiäre und weitere Zellkulturen sind solche Kulturen, die nach dem Anlegen einer Primärkultur einmal (sekundäre Kulturen), zweimal (tertiäre Kulturen) usw. "passagiert" wurden. Das Passagieren besteht im Umsetzen der Zellen von einem Kulturgefäß unter Hinzufügung von neuem Nährmedium und unter Verringerung der Zellkonzentration per ml Nährmedium in ein neues Kulturgefäß. Hierfür werden die Zellen in der Regel als Einzelzellsuspensionen aufbereitet.

Zellen werden als "diploid" bezeichnet, wenn sie in Kultur den normalen, unveränderten, artspezifischen Chromosomensatz haben und behalten. Selbstverständlich sind auch alle normalen Zellen im Organismus (bis auf einige Ausnahmen, wie bestimmte Zellen der Leber) diploid.

Abweichungen vom normalen, artspezifischen Chromosomensatz können zu heteroploiden bzw. aneuploiden und polyploiden Zellen führen. Die Unterscheidung zwischen beiden Begriffen ist unscharf. Aneuploide Zustände in Zellen sind gegeben, wenn beispielsweise ein Chromosom mehr oder weniger vorhanden ist. Ein Beispiel hierfür ist der Chromosomensatz mongoloider Menschen, der 47 Chromosomen anstatt 46, wie normalerweise beim Menschen vorhanden, umfasst. Ebenso kann der Verlust eines Chromosoms zur Aneuploidie führen: Menschen mit dem sogenannten Turner-Syndrom haben nur 45 Chromosomen, da ein X- (= Geschlechtschromosom) Chromosom fehlt.

Als permanente Zellinie wird bezeichnet, wenn aus einer Primärkultur über mehrere bis viele Passagen (in der Regel bis zu 70) die Zellkultur dann "unsterblich" geworden ist. Solche Zellkulturen werden als "Zellinien" bezeichnet, was ausdrückt, dass sie regelmässig passagiert werden können und sich in gleichen Zeiteinheiten (Tage bis Wochen) entsprechend gleichförmig vermehren können. Solche Zellinien sind weithin gebräuchlich in der biotechnologischen Forschung und Anwendungstechnik zur Herstellung von Bioprodukten. Sie werden in z.T. grossen Nährmediumvolumina gezüchtet, d.h. es wird damit eine Massenproduktion von Zellen betrieben.

Unter transformierten Zellen bzw. Zellinien versteht man Zellen und auch Zellinien, bei denen der Chromosomensatz bleibend vom diploiden Normalzustand abweicht. Es handelt sich hierbei um heteroploide bzw. aneuploide und polyploide Zellen. Hinzu kommt, dass transformierte (oder mutierte) Zellen auch morphologisch andere Erscheinungsformen haben als ihre Ausgangszellen im Organismus bzw. in der Primärkultur. Permanente Zellinien sind somit gegenüber ihrem Ursprungsgewebe/ -organ auch als "transformiert" anzusehen.

Adhärente Zellen: Viele Zelltypen, die ausserhalb des Organismus als Primärkulturen (oder folgende) und als permanente Zellinien gehalten und vermehrt werden, müssen dabei fest auf der Oberfläche des Kulturgefässes oder eines festen Trägers anhaften, um lebens- und vermehrungsfähig zu bleiben.

Unter die üblichen, d.h. nicht-zellspezifischen Nährmedien fallen die meisten im Handel befindlichen Nährmedien mit Bezeichnungen wie
- Minimal Essential Medium (H. Eagle - MEM, siehe Science 130 (1959), 432;
- Modified Eagle's Medium (M.D. Daniel und Mitarb.), siehe Proc. Soc. Exper. Biol. Med. 127 (März 1969);
- Dulbecco's Modified Eagles's Medium -DMEM, siehe Virology 8 (1959), 396 und 12 (1960), 185-196, Tissue Culture Standards Committee, In Vitro 6 Nr. 3, 93;
- McCoy"s 5a Medium - McCoy 5a, siehe Proc. Soc. Exper. Biol. Med. 100 (1959), 115-118;
- Leibovitz Medium - L-15, siehe Am. J. Hyg. 78 (1963), 173-180;
- Nährmedium F-10 und F-12 (R.G. Ham), siehe Exp. Cell Res. 29 (1963), 515-526 und Proc. Natl. Acad, Sci. 53 (1965), 288-293;
- Weymouth's Medium, siehe J. Natl, Cancer Inst. 22 (1959), 1003-1017;
- Roswell Park Memorial Institute 1640 - RPMI 1640 (G.E. Moore und Mitarb.), siehe J. Amer. Med. Assoc. 199 (1967), 519-524;
- Grace's Medium (T.D.C. Grace), siehe Nature 195 (1962), 788-789.

Zellspezifische Nährmedien sind Nährmedien, die für bestimmte Primärkulturen, Zellstämme oder Zellinien hergestellt wurden. Im einzelnen sind sie in bezug auf ihre Qualität nur zu beschreiben, wenn gleichzeitig die zugehörige Zellinie bzw. der zugehörige Zelltyp beschrieben wird. Einige dieser zellspezifischen Nährmedien lassen auch zu, dass mit ihnen andere Zellinien gezüchtet werden können, in der Regel jedoch mit weit geringerer Effizienz. Zellspezifische Nährmedien sind in der Regel Entwicklungen einzelner Laboratorien, die sowohl das Nährmedium auf die zu konservierenden oder gewünschten Zelleigenschaften angepasst haben, wie umgekehrt Zell-Selektion in der kultivierten Zellpopulation unter Einwirkung des spezifischen Nährmediums beschrieben haben. Hierbei selektionieren jene Zellen, die sich an die Nährmediumzusammensetzung und die übrigen Milieubedingungen adaptieren und überleben.

Bei der Durchführung des Verfahrens können die Zellen sowohl in immobilisiertem Zustand als auch frei flottierend gezüchtet bzw. gehalten werden.

Immobilisierte Zellen sind solche, die auf der Wand eines Kulturgefässes fest anhaften. Hinzugerechnet werden auch solche, die in von Nährmedium durchströmten Röhren- oder Kapillarsystemen auf der Innen- oder Außenwand der Röhren oder Kapillaren (Glas, Kohlefaser, Keramik) anhaften und sich vermehren. Die Vermehrung solcher Zellen kommt weitgehend zum Stillstand, wenn der zur Verfügung stehende Platz auf der Wandfläche von Zellen lückenlos besetzt wird. Es tritt dann die sogenannte "Kontakt-Inhibition" ein. Sie bewirkt, dass die Zellen sich über längere Zeiträume (Tage bis viele Wochen) nicht mehr vermehren, jedoch in das Nährmedium, das auch von einem geschlossenen Pumpsystem langsam und kontinuierlich vorbeigeführt werden kann, ihre Stoffwechselprodukte oder ihre über gentechnologische Massnahmen induzierte Substanzproduktion abgeben.

Zu den flottierenden Zellen gehören alle Zellen, die ohne die Erfordernis des festen Anhaftens auf einem festen Träger wie einer Kulturgefäßwand im Nährmedium schwebend gehalten und vermehrt werden können. Sie werden vor allem benötigt für sogenannte Fermenterkulturen, dies sind Kesselkulturen bis zu vielen tausend Liter Inhalt, in denen solche Zellen frei flottierend vermehrt werden. Dabei wird das flüssige Nährmedium z.B. mechanisch und/oder durch Luftblasenkonvektion ständig umgewälzt. Eine Variation sind adhärente Zellen auf sogenannten "Microcarrier"-Kunststoff-Kügelchen (0,1 - 1mm Durchmesser), die in der Fermenterflüssigkeit frei flottieren.

Die nach dem neuen Verfahren in Massenkulturen vermehrten und gehaltenen tierischen Zellen weisen, im Gegensatz zu den entsprechenden Kulturen ohne Zusatz des erwähnten Hämodialysats , keine der eingangs beschriebenen Schä digungen auf bzw. ganz wesentlich verminderte Schädigungen.

Beispiele 1 und 2 - Einleitung

Der zuvor erwähnte Erfolg lässt sich in besonders eindrücklicher Weise am Beispiel der Kultivierung und Vermehrung von 3A1-Hybridom-Zellen zeigen, die einen monoklonalen Antikörper produzieren, sowie mit L-929-Zellen, einer permanenten Maus-Zellinie. Diese Zellen vermehren sich in serumarmem Nährmedium, d.h. ein Nährmedium mit nur 2% bzw. 1,5% fetalem Kälberserumzusatz (FCS = Fetal Calf Serum) anstelle von 10% FCS-Zusatz, bei Anwesenheit von 2% Hämodialysat wesentlich besser als unter Nährmediumbedingungen ohne Hämodialysatzusatz.

Auch Stoffwechselleistungen sind besser, wie durch die höhere Produktion von monoklonalem Antikörper ($\mu$g IgG/ml) durch 3A1-Zellen bei Anwesenheit von Hämodialysat in MEM + 2% FCS nachgewiesen werden kann. Das Hämodialysat vermindert somit schädliche Einflüsse der in vitro-Bedingungen auf die Zellqualität und -vitalität.

Darüber hinaus, wie in der Einleitung, bei der Erwähnung der möglichen Schädigungsarten chemischer Natur hervorgehoben wurde, bringt der bisher erforderliche Zusatz von tierischem Serum (foetales Serum oder Serum von neugeborenen oder auch von erwachsenen Tieren) die Einführung von fremden Eiweissen in die Zellkultur mit sich. Hieraus ergibt sich bei der Gewinnung von biologischen Produkten die Notwendigkeit von aufwendigen Reinigungsoperationen. Nun macht der Zusatz des Hämodialysats anstelle von Serum diese Erfordernis überflüssig, wenn das Serum vollständig ersetzt werden kann, er vermindert den finanziellen, apparativen und personellen Aufwand für Reinigungsschritte in dem Masse, in dem das Serum teilweise ersetzt werden kann.

Beispiel 1 - Methodik

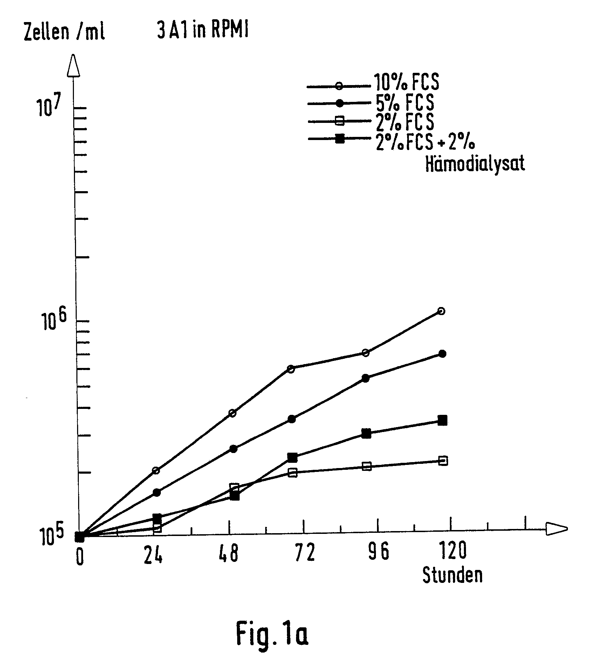

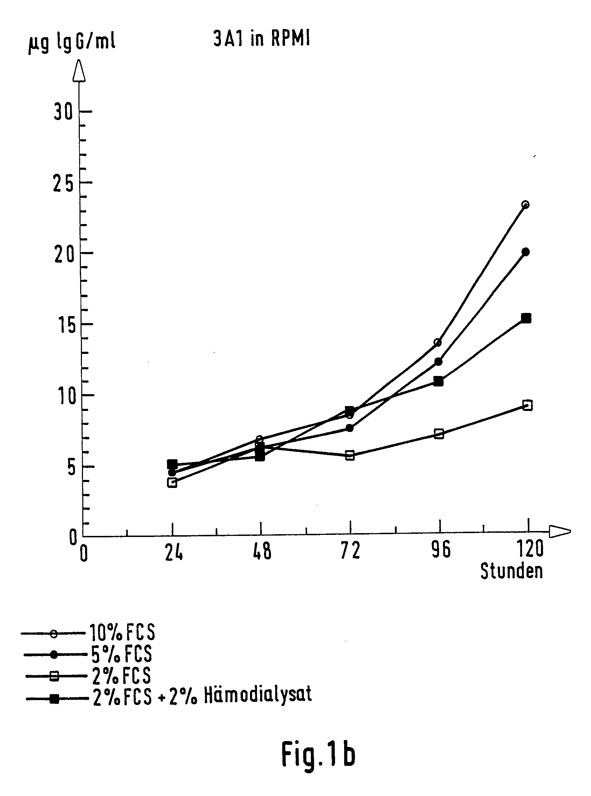

Zellen der Hybridomlinie 3A1 wurden als Stammkulturen in dem RPMI-Nährmedium oder MEM-Nährmedium mit einem Zusatz von 10% foetalem Kälberserum in Kunststoffflaschen für Zellkulturen (180 cm$^2$) gehalten. Von diesen Stammkulturen wurden mit einer Konzentration von 10$^5$ Zellen/ml 800 ml in eines 2000 ml Spinnerflasche gegeben und mit einem Magnetrührer bei ca. 50 Umdrehungen pro Minute gerührt. Den Nährmedien wurden nur 2% fetales Kälberserum (FCS) zugesetzt, d.h. der Serumzusatz war um 80% reduziert im Vergleich zur Ausgangskultur. Zu diesen Nährmedien wurden 2% Hämodialysat zugesetzt. Die Abbildungen 1a und 1b zeigen die Wirkung des Hämodialysatzusatzes im RPMI-Nährmedium. Innerhalb 5 Tagen vermehren sich die Zellen um einen Faktor von 1,5 besser (Fig. 1a) und produzieren um einen Faktor 1,6 mehr (Fig. 1b) monoklonale Antikörper im Vergleich zur Kultur mit 2% FCS-Zusatz.

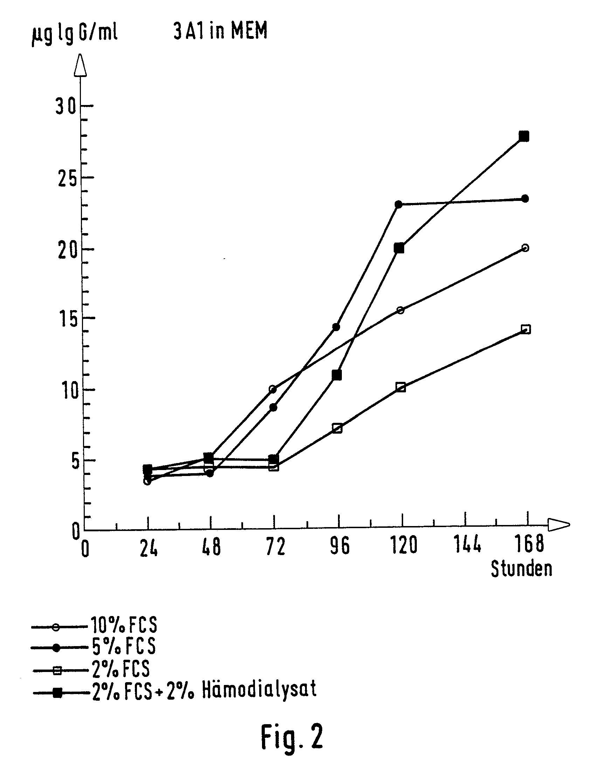

In Fig. 2 wird in einem Experiment mit dem MEM-Nährmedium gezeigt, dass die Produktion der monoklonalen Antikörper um einen Faktor 2,0 höher ist als in der Kultur mit nur 2% FCS. Auf den Abbildungen 1 und 2 sind zum Vergleich die Werte für Zellen in Nährmedien mit 5% und 10% FCS-Zusatz mit angegeben.

Beispiel 2 - Methodik

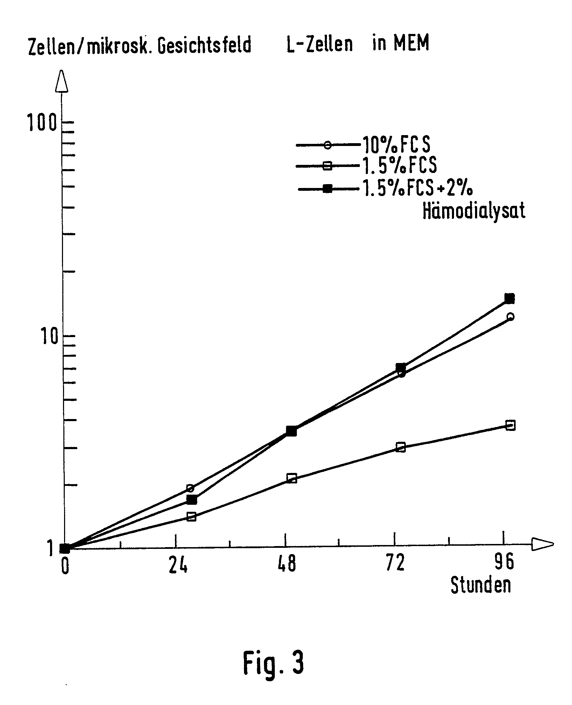

In Abbildung 3 wird an Zellen der permanenten Zellinie L-929 der Maus die Wirkung von 2% Hämodialysatzusatz zum MEM-Nährmedium + 1,5% FCS gezeigt. Das Experiment wurde in Zellkulturflaschen aus Kunststoff (175 cm$^2$) durchgeführt. Als Mass für die Zellvermehrung wurde der Mittelwert der Zunahme an Zellen in 20 mikroskopischen Gesichtsfeldern gewählt; für das Experiment wurden nur Gesichtsfelder mit je 1 vitalen Zelle ausgewählt.

Die Einsaat erfolgte aus Stammkulturen in MEM + 10% FCS mit einer Konzentration von 5 x 10$^2$ Zellen/ml. Diese niedrige Zellkonzentration wurde gewählt, um die Reaktion einzelner Zellen auf die Wirkung des Hämodialysats besser beurteilen zu können. Wie die Werte zeigen (Fig. 3), führt der Zusatz von 2% Hämondialysat zu einer um einen Faktor 4,0 erhöhten Zellvermehrung im Vergleich zu Zellen in MEM + 1,5% FCS.

Die durch das Hämodialysat bewirkte höhere Zellvermehrung wird entsprechend auch in L-929-Kulturen mit Einsaatdichten von 10$^5$ Zellen/ml erreicht. In 175 cm$^2$-Kunststoffkulturflaschen wer-

den folgende Zellzahlen pro ml am 4. Tag nach der Einsaat erreicht:

MEM + 10% FCS     $2,16 \times 10^6$
MEM + 1,5% FCS     $6,48 \times 10^5$
MEM + 1,5% FCS + 2% Hämodialysat     $2,05 \times 10^6$

### Beispiele 3 und 4 - Einleitung

Für viele Zellkulturen dienen $CO_2$ und Natriumbicarbonat nicht nur im Gleichgewicht stehend zur Pufferung des Mediums, sondern sie sind auch für den Zellstoffwechsel erforderlich.
(Swim and Parker, 1958, The Role of Carbon Dioxide as an essential Nutrient for six permanent Strains of Fibroblasts, J. Biophys. Biochem. Cytol. 4, 525-528; Geyer and Chang, 1958, Bicarbonate as an Essential for human cells in Vitro, Arch. Biochem. Biophys. 73, 500-506;
Runyan and Geyer, 1967, Partial Replacement of $CO_2$ in strain L and HeLa Cultures, Proc. Soc. Exp. Biol. Med. 125, 1301-1304).

Deshalb werden Zellkulturen in der Regel unter atmosphärischen Bedingungen mit Zusatz von 5 bit 10% $CO_2$ in einem Medium, das 0,25 bis 5 g/Liter $NaHCO_3$ enthält, gezüchtet. Fällt z.B. die $CO_2$-Zufuhr aus oder zersetzt sich das Carbonat durch Wärme oder Säureeinwirkung, so werden die Zellkulturen geschädigt. Beispiel 3 und 4 belegen, dass in Massenkulturen der Zusatz des Hämodialysates, das selbst höchstens geringe Mengen an Carbonat enthält (< 100 mg/Liter), diese schädigenden Auswirkungen wesentlich zu vermindern vermag.

### Beispiel 3 - Methodik

Alle Arbeiten erfolgen unter sterilen Bedingungen in einer Sterilbox.

(1) LM-Fibroblasten (Bindegwebe Maus, sekundäre Zellinie; ATCC CCL 1.2 L-M) werden in einer Kunststoffflasche mit einer Kulturfläche von 75 $cm^2$ mit 20 ml Minimum Essential Medium Eagle (2,2 g/l Natriumbicarbonat) mit 5% foetalem Kälberserum (FCS) gezüchtet. Die Einsaat beträgt 50'000 Zellen pro Kulturflasche. Die Inkubation erfolgt in einem $CO_2$-Brutschrank bei 37°C und 5% $CO_2$. Nach viertägiger Inkubation wird das Medium erneuert. Nach 7 Tagen werden die Zellen aufgearbeitet (2) und die Testkulturen angesetzt (A, B).

(2) Das Medium wird vollständig entfernt. Die Zellen werden anschliessend mit 5 ml 0,25% Trypsinlösung bedeckt und unter gelegentlichem leichtem Bewegen bei 37°C, bis sich der Zellrasen löst (ca. 5 Minuten), inkubiert. Die 5 ml-Zellsuspensionen werden mit 7 ml Medium mit FCS versetzt und während 10 Minuten bei 800 rpm (100 g) in einem konischen Reagenzglas mit Verschluss zentrifugiert. Der Ueberstand wird abdekantiert und die Fibroblasten in 5 ml Medium mit 5% FCS resuspendiert. Die Zellzahl dieser Suspension wird mittels Coulter Counter oder mittels Mikroskop nach Färbung mit Trypanblau bestimmt.

(3) Zwei 75 $cm^2$-Kulturflaschen werden mit je 500'000 Zellen dieser Suspension beimpft. Diese Flaschen enthalten folgende Medien:
A: 20 ml Minimum Essential Medium Eagle ohne Bicarbonat, mit 5% FCS;
B: 20 ml Minimum Essential Medium Eagle ohne Bicarbonat, mit 5% FCS und 4% Hämodialysat. Die Kulturen werden während 3 Tage bei 37°C im Brutschrank ohne $CO_2$ (die Deckel der Kulturflaschen werden geschlossen) inkubiert.

(4) Nach dreitägiger Inkubation werden die Zellen wie unter (2) gelöst und abzentrifugiert. Die Zellen werden dann in PBS (Phosphat Buffer Saline) resuspendiert und die Zellzahl bestimmt.
Gefunden werden pro Kulturflasche
- für die Kultur A    $1,2 \bullet 10^6$ Zellen,
- für die Kultur B    $2 \bullet 10^6$ Zellen.
Der Zusatz von 4% Hämodialysat in Kulturen der LM-Fibroblasten unter Carbonatentzug schützt die Zellen gegen Auswirkungen der Stress-Situation, wodurch sich eine um ca. 67% höhere Ausbeute an Zellen ergibt.

### Beispiel 4 - Methodik

(1) HEPM-Zellen (ATCC CRL 1486; Human Embryonic Palatal Mesenchyme) werden in Zellkultur - Rollerflaschen mit je einer Kulturfläche von 850 $cm^2$ mit 300 ml Minimum Essential Medium Eagle (mit 2,2 g/liter Natriumbicarbonat) mit 10% foetalem Kälberserum (FCS) - gezüchtet. Die Einsaat beträgt 2,5 Millionen Zellen pro Rollerflasche. Die Inkubation erfolgt bei 37°C in einem Rollerapparat (Bellco Glass. Inc., Vineland, NJ, USA) mit ca. 1 rpm Geschwindigkeit. Nach dreitägiger Inkubation wird das Medium erneuert. Nach 5 bis 7 Tagen werden die Zellen aufgearbeitet (2) und die Testkulturen angesetzt (3).

(2) Das Medium wird vollständig entfernt. Die Rollerflasche wird mit 25 ml Trypsin-Versen-Lösung (Natriumchlorid 8 g/Liter, Kaliumchlorid, 0,4 g/Liter, Glucose 1,1 g/Liter, $NaHCO_3$ 0,58 g/Liter, Trypsin 0,025%, Ethylendiamintetraessigsäure 0,2 g/Liter) gespült und anschliessend mit 25 ml Trypsin-Versen-Lösung auf dem Rollerapparat bei 37°C inkubiert. Wenn der Zellrasen gelöst ist (nach ca. 4 Minuten) wird die Zellsuspension mit 25 ml Medium mit FCS versetzt und während 10 Minuten in einem konischen Reagenzglas mit Verschluss zentrifugiert. Der Ueberstand wird abdekantiert und die Zellen in 10 ml Medium mit 10% FCS resuspendiert. Die Zellzahl dieser Suspension wird mittels Coulter Counter oder mittels Mikroskop nach Trypanblaufärbung bestimmt.

(3) Rollerflaschen (A und B) werden mit folgendem Medium mit je 2,5 Millionen Zellen beimpft und gut verschlossen.

     A (geschädigt, ohne Hämodialysat): 300 ml Minimum Essential Medium Eagle mit Earle's Salz ohne Bicarbonat und mit 10% FCS,

     B (geschädigt, mit Hämodialysat): 300 ml Minimum Essential Medium Eagle mit Earle's Salz ohne Bicarbonat, mit 10% FCS und 4% Hämodialysat.

4. Nach dreitägiger Inkubation bei 37°C werden die Zellen wie unter (2) gelöst und abzentrifugiert. Die Zellen werden in Medium mit 10% FCS resuspendiert und die Zellzahl bestimmt.

Gefunden werden pro Flasche
- für die Kultur A    $3,2 \cdot 10^6$ Zellen,
- für die Kultur B    $5,6 \cdot 10^6$ Zellen.

Der Zusatz von 4% Hämodialysat in Kulturen der HEPM-Zellen unter Carbonatentzug und gleichzeitiger mechanisch erzeugter Schädigung schützt die Zellen gegen die Auswirkungen der Stress-Situation, wodurch sich eine um ca. 75% höhere Ausbeute an Zellen ergibt.

## Beispiel 5

Auf dieselbe Weise, wie im Beispiel 1 beschrieben wird, wird eine Kultur des Hybridomzellklons D1 im RPMI-Medium unter den normalen Bedingungen einer Stammkultur angelegt. In einem Spinner von 1 Liter Inhalt wird eine Suspensionskultur bei einer Geschwindigkeit von 50 bis 60 Umdrehungen/Minute, ohne Sauerstoffeintrag durchgeführt; das Medium enthält nur 2% foetales Kälberserum (FCS).

In einer unter identischen Züchtungsbedingungen geführten parallelen Kultur enthält das Medium ebenfalls nur 2% foetales Kälberserum (FCS), dazu aber 2% Hämodialysat.

Gemessen werden, wie in den vorangehenden Beispielen angegeben wird, einerseits die Anzahl der im Verlauf der Kultur entstandenen Zellen per ml Kulturmedium, andererseits die Menge der im Verlauf der Kultur produzierten monoklonalen Antikörper per ml; die Ausbeute an Antikörpern wird in µg Immunoglobulin G (IgG) per ml ausgedrückt.

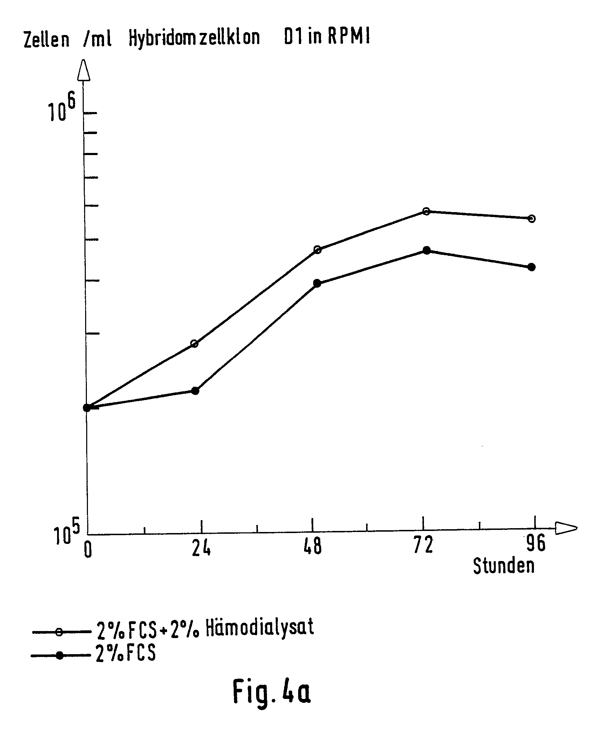

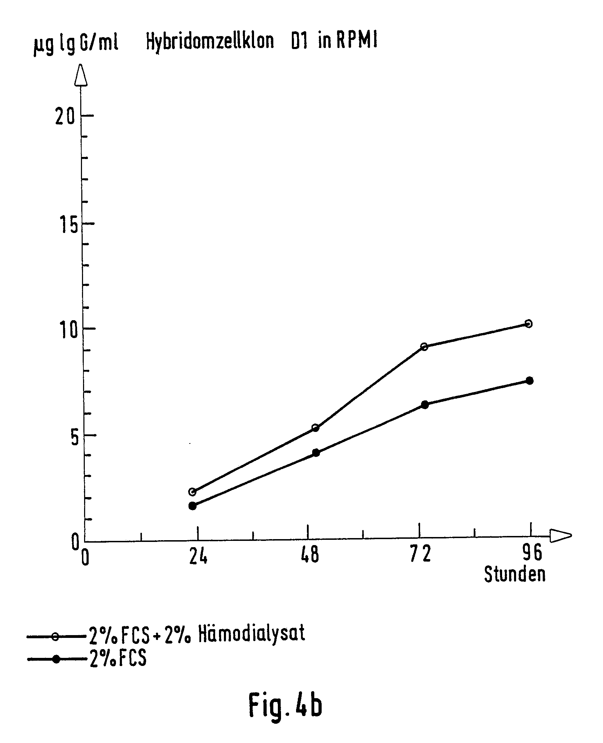

Aus den Fig. 4a und 4b ist die Wirkung eines Hämodialysat-Zusatzes von 2% (bezogen auf das Volumen des Kulturmediums) auf eine an FCS armen Kultur des Hybridomzellklons D1 klar erkennbar. Nach einer Züchtungsdauer von beispielsweise 72 Stunden bewirkt der Hämodialysat-Zusatz eine Steigerung der Antikörperproduktion um 48% gegenüber der FCS-armen Kultur ohne Zusatz und eine Steigerung der Zellenproduktion um einen Faktor von 1,3, was einer Steigerung der Zellpopulation um 25% und der Produktivität der einzelnen Zellen um etwa 14% entspricht. Die Auswirkungen der durch den an sich ungenügenden FCS-Gehalt des Mediums bedingten Stress-Situation werden durch einen Hämodialysat-Zusatz von nur 2% weitgehend vermieden.

## Beispiel 6

Es werden wie im beispiel 5 zwei parallele Kulturen des Hybridomzellklons D1 im RPMI-Medium durchgeführt, mit dem Unterschied allerdings, dass in beiden Kulturen ein Zusatz an foetalem Kälberserum (FCS) von 5% verwendet wird. Ein solcher Zusatz wird für dieses Kultursystem als optimal betrachtet, sei es im Hinblick auf die Zellenproduktion oder im Hinblick auf die mengenmässige Erzeugung von monoklonalen Antikörpern. In der zweiten Kultur wird ein Hämodialysat-Zusatz von 2% (bezogen auf das Volumen des Mediums) verwendet.

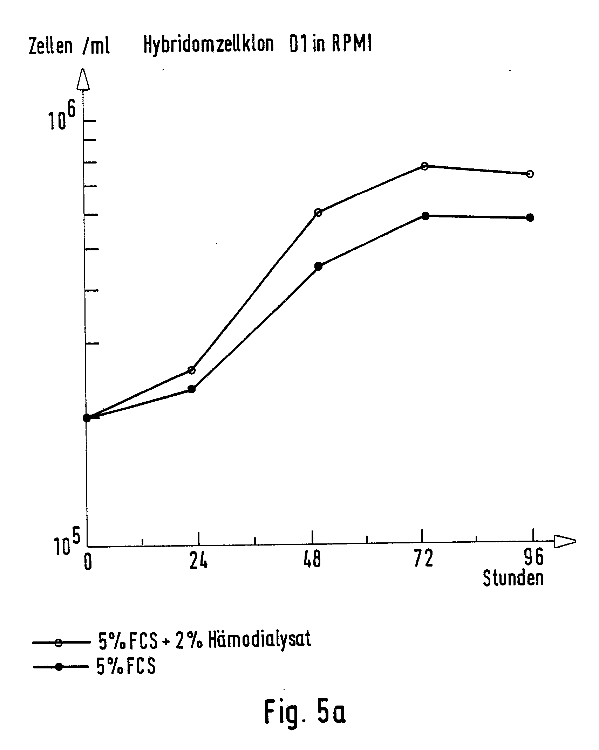

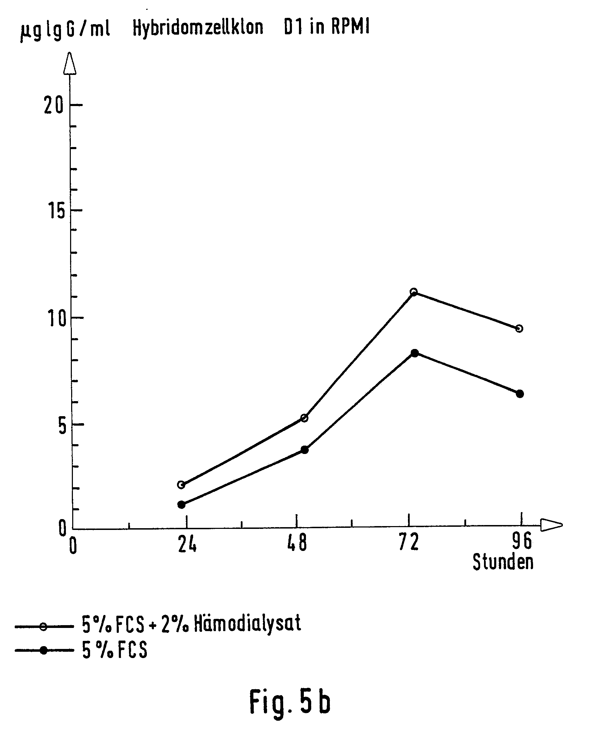

Die Abbildungen in Fig. 5a und 5b zeigen den Verlauf der beiden Kulturen in bezug auf die Produktion von Zellen (Fig. 5a) und von Antikörpern (Fig. 5b).

Obschon die Züchtung bei einem 5% FCS-Zusatz als für dieses System voll befriedigend gilt, erbringt der Hämodialysat-Zusatz dennoch beispielsweise nach einer Züchtungsdauer von 72 Stunden eine Erhöhung der Ausbeute an Antikörpern um etwa 35% und ein erhöhung der Produktivität der einzelnen Zelle um etwa 15% gegenüber der Kultur ohne diesen Zusatz. Diese Erhöhung erklärt sich durch die Schutzwirkung des Hämodialysates gegen die Auswirkungen des durch das Rühren der Kultur bewirkten zellulären Stresses.

## Patentansprüche

1. Verfahren zur Vermehrung und Haltung, als Massenzellkulturen, von tierischen
- Geweben oder Organexplantaten,
- primären, sekundären, tertiären oder weiteren Zellkulturen,
- diploiden oder heteroploiden bzw. aneuploiden Zellstämmen und Zellinien oder
- permanenten, gegebenenfalls transformierten Zellinien, mit den Merkmalen von adhärentem oder nicht-adhärentem Wachstum, in einem üblichen oder speziellen Kulturmedium, dadurch gekennzeichnet, dass man der Kultur zur Vermeidung von Schädigungen aller Art, welche während der Massenkultivation durch die in vitro-Situation verursacht werden können, ein von makromolekularen Substanzen freies Hämodialysat oder Ultrafiltrat aus Kälberblut, dessen Komponenten Molekulargewichte unter 10'000 habe, zusetzt.

2. Verfahren nach Anspruch 1, dadurch gekennzeichnet, dass als Hämodialysat oder Ultrafiltrat ein solches verwendet wird, zu, dessen Herstellung etwa das 6-fache Volumen von Kälberblut eingesetzt wird.

3. Verfahren nach Anspruch 1, dadurch gekennzeichnet, dass der Kultur mindestens etwa 0,5 Volumen-% Hämodialysat oder Ultrafiltrat, bezogen auf das Volumen der Kulturflüssigkeit, zugesetzt werden.

4. Verfahren nach Anspruch 3, dadurch gekennzeichnet, dass der Kultur von etwa 0,5 bis etwa 4 Volumen-% Hämodialysat oder Ultrafiltrat, bezogen auf das Volumen der Kulturflüssigkeit, zugesetzt werden.

5. Verfahren nach Anspruch 1, dadurch gekennzeichnet, dass die Kultur immobilisiert oder frei flottierend durchgeführt wird.

Fig. 1a

Fig.1b

Fig. 2

Fig. 3

Zellen /ml Hybridomzellklon D1 in RPMI

$10^6$

$10^5$

0  24  48  72  96

Stunden

—o— 2% FCS+2% Hämodialysat
—•— 2% FCS

# Fig. 4a

Fig. 4b

Zellen /ml    Hybridomzellklon  D1 in RPMI

—o— 5% FCS + 2% Hämodialysat
—●— 5% FCS

# Fig. 5a

µg Ig G / ml    Hybridomzellklon   D1 in RPMI

—○— 5% FCS + 2% Hämodialysat
—●— 5% FCS

Fig. 5 b